Europäisches Patentamt

European Patent Office

Office européen des brevets

(11) Veröffentlichungsnummer: **0 147 516**
**B1**

## EUROPÄISCHE PATENTSCHRIFT

(45) Veröffentlichungstag der Patentschrift:
**11.05.88**

(21) Anmeldenummer: **84108319.9**

(22) Anmeldetag: **14.07.84**

(51) Int. Cl.⁴: **A 61 M 16/04**

(54) **Vorrichtung zur Erfassung und Auswertung des Druckes in der Ballonmanschette eines geschlossenen Trachealtubus.**

(30) Priorität: **29.07.83 DE 3327342**

(43) Veröffentlichungstag der Anmeldung:
**10.07.85 Patentblatt 85/28**

(45) Bekanntmachung des Hinweises auf die Patenterteilung:
**11.05.88 Patentblatt 88/19**

(84) Benannte Vertragsstaaten:
**FR GB NL**

(56) Entgegenhaltungen:
**DE - A - 3 204 110**
**US - A - 4 285 340**

(73) Patentinhaber: **Drägerwerk Aktiengesellschaft,
Moislinger Allee 53-55, D-2400 Lübeck 1 (DE)**

(72) Erfinder: **Weerda, Gerd-Hilko, Prof. Dr. Dr.,
Kastelbergstrasse 1, D-7812 Bad Krozingen (DE)**
Erfinder: **Pedersen, Peter, Rastatter Strasse 8,
D-7800 Freiburg (DE)**

**Beschreibung**

Die Erfindung betrifft eine Vorrichtung zur Erfassung und Auswertung des Druckes in der Ballonmanschette eines geschlossenen Trachealtubus mit einem an die Aufblaskanüle der Ballonmanschette angeschlossenen Druckwandler, dem ein Anzeigeinstrument nachgeschaltet ist und mit einer Steuerschaltung zum Entblocken der Ballonmanschette beim Überschreiten eines zulässigen Druckbereichs.

Bei der künstlichen Beatmung eines Patienten mit einem geschlossenen Trachealtubus, wie er aus der älteren Patentanmeldung P 32 04 10.1-35 bekannt ist, und einem für die geschlossene Beatmungstechnik ausgebildeten Respirator wird der Trachealtubus in der Trachea so appliziert, daß dessen offenes Ende zur Carina und den Lungen zeigt. Durch Aufblasen einer den Tubus umgebenden Ballonmanschette wird der Tubus an der Tracheawandung fixiert und dichtet zusammen mit dem geräteseitigen Ende des Tubus den Lungenraum gegen den Larynx ab. Die Beatmung bei einem solchen Tubus erfolgt über einen dünnen Beatmungsschlauch, durch den Atemgas eingeblasen und abgesaugt wird. Eine ebenfalls in den Tubus hineinragende Druckmeßkanüle dient zum Messen des intratrachealen Beatmungsdruckes und damit zur Steuerung des Respirators in der Weise, daß in den Lungen die physiologisch erforderlichen Atemmuster entstehen.

Wenn in das offene Ende der Druckmeßkanüle Sekrete aus dem Tracheal- und Lungenbereich des Patienten eintreten, besteht die Gefahr von Verstopfungen und damit das Risiko der Verfälschung oder des Ausbleibens der Druckmessung. Solche Fehlmessungen führen aber zu einer fehlerhaften Respirationssteuerung und damit zu einer falschen Beatmung.

In der US-A 4 285 340 ist eine Vorrichtung zur Regelung des Druckes in der Ballonmanschette eines Trachealtubus angegeben, die die Merkmale nach dem Oberbegriff des Anspruchs 1 aufweist. Die Ballonmanschette ist an ein ansteuerbares Ventil angeschlossen, welches zur Füllung mit einer Gasquelle und zur Entleerung der Manschette mit der Umgebung verbindbar ist. Während des Einatemzyklus eines angeschlossenen Beatmungsgerätes wird durch entsprechende Steuersignale die Ballonmanschette unter Druck gesetzt, damit ein enges Anliegen an der Tracheainnenwand ein leckfreies Erzeugen eines Überdrucks in der Lunge des Patienten gewährt ist. Beim nachfolgenden Ausatemzyklus wird durch Ansteuerung des Ventils die Manschette zumindest teilweise entlastet. Somit kann während der Ausatmung entweder ein positiver Überdruck in der Lunge des Patienten aufrecht erhalten bleiben, wenn die Manschette nur teilweise druckentlastet ist, oder es wird bei einer vollständigen Druckentlastung Atmosphärendruck in der Lunge hergestellt. Die augenblicklichen Druckwerte in der Manschette werden mit einem Referenzwert verglichen, und die Differenz beider Werte als Steuergröße für die gewünschte Druckentlastung der Manschette herangezogen.

Der Manschettendruck ist über ein Anzeigeinstrument ablesbar.

Mit der bekannten Vorrichtung wird lediglich eine Steuerung des Manschettendruckes, gekoppelt an den Einatem- und Ausatemzyklus, möglich.

Der Erfindung liegt die Aufgabe zugrunde, eine Vorrichtung der eingangs genannten Art zu schaffen, bei der die Gefahr einer falschen Beatmung infolge einer Verstopfung der Druckmeßkanüle vermieden ist.

Diese Aufgabe wird bei einer Vorrichtung der eingangs genannten Art durch die kennzeichnenden Merkmale des Anspruchs 1 gelöst.

Mit der erfindungsgemäßen Vorrichtung kann einerseits eine fehlerhafte Abdichtung infolge zu geringen Manschettendruckes oder eines Manschettenleckes und andererseits auch eine zu schnelle oder zu langsame Änderung der Atemdruckschwankungen erfaßt werden. Derartige Fehlerzustände lösen ein Entblocken der Ballonmanschette sowie ein Umschalten des Respirators aus. Statt einer geschlossenen Beatmung unter Berücksichtigung der von der Druckmeßkanüle erfaßten Ist-Werte des Intratrachealdruckes erfolgt dann eine offene Injektbeatmung ohne Berücksichtigung des Trachealdruckes und der Durchflußmengen. Nach der Beseitigung der Störung kann die Ballonmanschette wieder aufgeblasen und der Respirator auf die Betriebsart «Dauerbeatmung» umgeschaltet werden.

Der zweite Integrator verhindert, daß kurzzeitige Störungen durch Bewegungen des Patienten bereits zu einem Entblocken des Trachealtubus und Umschalten des Respirators führen. Derartige kurze Störungen werden nur optisch und akustisch angezeigt, wenn sie sich innerhalb einer durch die Zeitkonstante des Integrators vorgegebenen Zeit nicht mehrfach wiederholen.

Zweckmäßige Weiterbildungen des Gegenstandes der Erfindung sind in den Unteransprüchen gekennzeichnet.

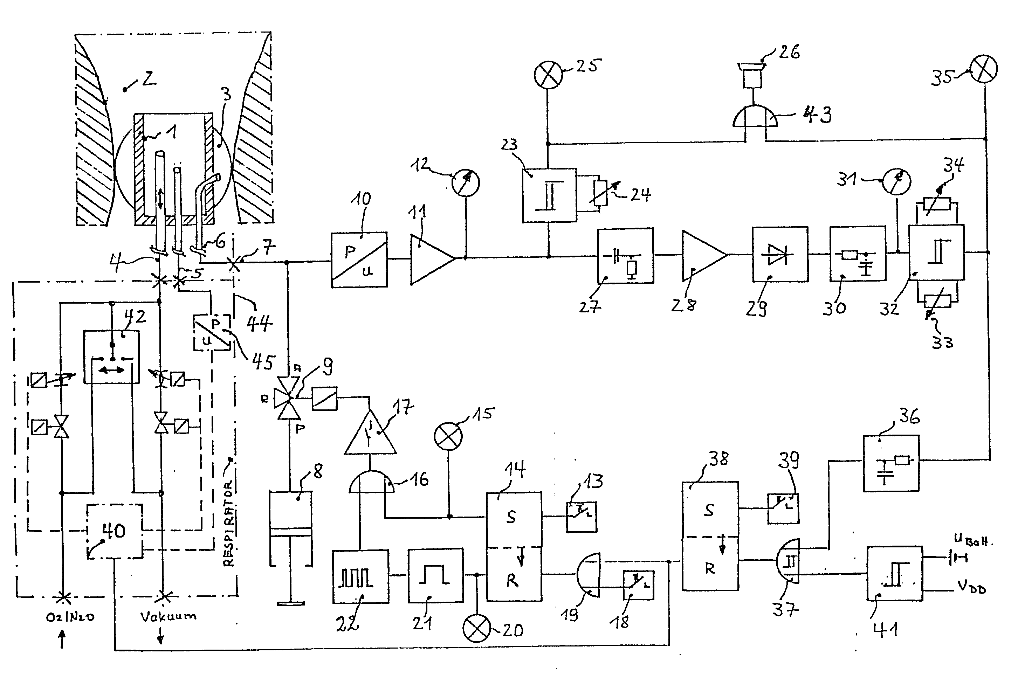

In der Zeichnung ist ein Ausführungsbeispiel der Erfindung dargestellt und zeigt die Vorrichtung zur Erfassung und Auswertung des Druckes in der Ballonmanschette zusammen mit einem Teil des Respirators in einem Blockschaltbild.

Der in der Zeichnung schematisch dargestellte Trachealtubus 1 ist so in der Trachea eines Patienten zur künstlichen Beatmung eingesetzt, daß das offene Ende des Tubus zum Intratrachealraum 2 weist. Mit einer Ballonmanschette 3, die aus einem dünnwandigen weichen Gummimantel besteht, der jeweils an den beiden Enden radial mit dem Tubuskörper verschweißt ist und so einen aufblasbaren Ring bildet, ist der Trachealtubus 1 an der Tracheawandung durch Aufblasen fixiert und dichtet zusammen mit dem geräteseitigen Verschluß des Trachealtubus 1 den Lungenraum gegen den Larynx ab.

Die Beatmung erfolgt über einen dünnen Beatmungsschlauch 4, der in den freien Raum des Tubuskörpers hineinragt. Die Beatmungskanüle 4 ist mit einem in der Zeichnung schematisch dargestellten Respirator 44 verbunden. Zur Steuerung des Respirators 44 ist dieser über eine Druckmeßkanüle 5 mit dem Intratrachealraum 2 verbunden. Die Druckmeßkanüle 5 endet an einem Druckaufnehmer 45, der den intratrachealen Beatmungsdruck mißt und die Steuerung des Respirators 44 so regelt, daß in den Lungen die physiologisch erforderlichen Atemmuster entstehen.

Das Innere der Ballonmanschette 3 ist über eine Aufblaskanüle 6 mit einem Pumpenanschluß 7 verbunden, über den mit Hilfe einer Pumpe 8, die beispielsweise eine Injektionsspritze sein kann, bei geöffnetem 3/2-Wege-Magnetventil 9 Luft in die Ballonmanschette 3 so lange gepumpt werden kann, bis die Ballonmanschette 3 dicht gegen die Tracheawand anliegt und somit zu einem geschlossenen Beatmungssystem führt.

Der in der Ballonmanschette 3 herrschende Druck wird mit Hilfe eines Druck/Spannungs-Wandlers 10 erfaßt. Die dem jeweiligen Druck entsprechende elektrische Spannung gelangt über einen Verstärker 11 zu einem Anzeigeinstrument 12 für die Anzeige des statischen Druckes in der Ballonmanschette 3. Auf diese Weise kann angezeigt werden, ob der für ein Anliegen der Ballonmanschette 3 an die Tracheawand erforderliche Druck vorhanden ist.

Zur Abdichtung gegen die Pumpe wird das Magnetventil 9 durch Betätigen einer Taste 13 geschlossen, mit der ein R-S-Flip-Flop 14 eingesetzt wird. Der Ausgang des R-S-Flip-Flops 14 ist einerseits mit einer optischen Statusanzeigeeinrichtung 15 und andererseits mit dem ersten Eingang eines ODER-Gatters 16 verbunden. Der Ausgang des ODER-Gatters 16 ist an den Eingang eines Leistungsschalters 17 angeschlossen, durch den das Magnetventil 9 betätigbar ist.

Durch Betätigen einer Taste 18, die über ein ODER-Gatter 19 mit dem Rücksetzeingang des R-S-Flip-Flops 14 in Verbindung steht, kann das Magnetventil 9 geöffnet werden, so daß der Druck in der Ballonmanschette 3 entweicht und um die Ballonmanschette 3 ein Ringraum freigegeben wird.

Der Rücksetzausgang des R-S-Flip-Flops 14 ist einerseits mit einer optischen Statusanzeigeeinrichtung 20 und andererseits mit einer monostabilen Kippstufe 21 verbunden, durch die beim Rücksetzen des R-S-Flip-Flops 14 ein etwa eine Sekunde dauernder Impuls erzeugt wird. Mit dem Ausgang der monostabilen Kippstufe 21 ist ein Rechteckimpulsgenerator 22 verbunden, der während der Dauer des durch die monostabile Kippstufe 21 erzeugten Impulses etwa 15 Einzelimpulse zur Steuerung des Magnetventils 9 über das ODER-Gatter 16 und den Leistungsschalter 17 erzeugt. Auf diese Weise wird das 3/2-Wege-Magnetventil 9 etwa 15 mal kurz auf- und zugeschaltet, so daß über dem freien R-Ausgang die Luft aus der Ballonmanschette 3 in den freien Raum entweichen kann.

Wenn nach dem Aufpumpen der Ballonmanschette 3 und Betätigen des Tasters 13 das Magnetventil 9 geschlossen ist, wird der statische Druck in der Ballonmanschette 3 mit Hilfe eines ersten Grenzwertschalters 23 überwacht, dessen Eingang mit dem Ausgang des Verstärkers 11 verbunden ist. Die Schaltschwelle des Grenzwertschalters 23 kann mit Hilfe eines Grenzwertstellers 24 zur Einstellung des minimalen statischen Druckes zwischen 0 und 100 mbar gewählt werden. Fällt der Manschettendruck und damit die Ausgangsspannung des Verstärkers 11 unter die eingestellte Schwelle, steuert der Grenzwertschalter 23 eine optische Anzeigeeinrichtung 25 und über ein ODER-Gatter 43 zusätzlich eine akustische Anzeigeeinrichtung 26 an. Wenn das akustische Warnsignal ertönt und gleichzeitig die optische Anzeigeeinrichtung 25 aufleuchtet, wird der Bedienungsperson auf diese Weise mitgeteilt, daß ein zu geringer Manschettendruck vorliegt, der beispielsweise durch ein Manschettenleck verursacht worden sein kann.

Der Ausgang des Verstärkers 11 ist weiterhin mit dem Eingang eines Differenziergliedes 27 verbunden, das die dem statischen Manschettendruck überlagerten Atemdruckschwankungen p(t) von dem statischen Manschettendruck abtrennt. Das Spannungssignal entspricht jetzt der zeitlichen Änderung der Atemdruckschwankungen, dp(t)/dt, welches einem Verstärker 28 zugeführt wird. Von dort aus gelangt es zu einem Doppelweggleichrichter 29, dessen Signal in einem ersten Integrator 30 aufsummiert wird. Diese Aufsummierung erfolgt um so schneller, je häufiger bzw. schneller die zeitliche Änderung der Atemdruckschwankungen in der Ballonmanschette 3 erfolgt. Die Spannung am Anzeigeinstrument 31 entspricht somit der mittleren Änderungsrate der differenzierten Atemdruckschwankungen, d/dt (dp[t]/dt).

Der Ausgang des ersten Integrators 30 ist weiterhin mit dem Eingang eines zweiten Grenzwertschalters 32 verbunden, dem ein Grenzwertsteller 33 für die langsamste Änderung der Atemdruckschwankungen und ein Grenzwertsteller 34 für die schnellste Änderung der Atemdruckschwankungen zugeordnet sind. Bei Überschreitungen bzw. Unterschreitungen der mit den Grenzwertstellern 33 und 34 eingestellten Grenzwerte schaltet der zweite Grenzwertschalter 32 durch und verursacht eine optische Anzeige über eine optische Anzeigeeinrichtung 35 sowie eine akustische Anzeige über die bereis oben erwähnte akustische Anzeigeeinrichtung 26.

Das Ausgangssignal des zweiten Grenzwertschalters 32 gelangt außerdem zu einem zweiten Integrator 36. Wenn die Häufigkeit oder die Dauer der vom zweiten Grenzwertschalter 32 abgegebenen Signale einen von der Zeitkonstante des zweiten Integrators 36 abhängenden Wert übersteigen, wird durch das aufintegrierte Signal ein Durchschalten eines ODER-Gatters 37 mit Schmitt-Trigger-Verhalten ausgelöst. Auf diese Weise wird bei Überschreitung einer vorgegebenen Störzeit durch das Ausgangssignal des ODER-Gatters 37 ein Rücksetzen eines zweiten R-S-Flip-Flops 38 bewirkt, das mit Hilfe eines Tasters 39 zur Dauerbeatmung gesetzt worden ist.

Bei kurzen Störungen leuchtet die optische Fehleranzeigeeinrichtung 35 kurz auf, ohne daß das zweite R-S-Flip-Flop 38 zurückgesetzt wird. Erst wenn die Störung so lange dauert, daß die durch den zweiten Integrator 36 vorgegebene Störzeit überschritten wird, erfolgt ein Rücksetzen des zweiten R-S-Flip-Flops 38 und damit über das ODER-Gatter 19 ein Rücksetzen des ersten R-S-Flip-Flops 14, was ein Entblocken der Ballonmanschette 3 zur Folge hat.

Die Störungsmeldung durch Rücksetzen des zweiten R-S-Flip-Flops 38 wird, wie im Blockschaltbild erkennbar ist, auch an die Respiratorsteuerung 40 des Respirators 44 weitergeleitet, so daß eine Umschaltung von der Dauerbeatmung mit Einkopplung der IST-Werte vom Intratrachealdruck und Inspirationsflow für Regelzwecke auf eine Betriebsart zur Ein- bzw. Ausleitung der künstlichen Beatmung bewirkt wird. Hierbei arbeitet der Respirator 44 nach vorgegebenen Sollwerten, ohne daß der Trachealdruck und/oder der Gasflow als Meßgröße oder Steuergröße verwendet werden.

Nach dem Umschalten des Respirators 44 erfolgt die Beatmung wie bei der offenen Injektbeatmung, wobei der Trachealtubus 1 nicht mehr dicht in der Trachea sitzt. Die während dieser Beatmung entstehenden Überdrücke bzw. Unterdrücke werden durch den Ringzwischenraum zwischen Trachea und Trachealtubus 1 ausgeglichen.

Wenn die das Umschalten verursachende Störung beseitigt worden ist, kann die Ballonmanschette 3 wieder aufgeblasen und der Respirator 44 wieder auf die Betriebsart «Dauerbeatmung» umgeschaltet werden.

Zur Erkennung eines Stromausfalls ist ein Betriebsstromüberwachungsschaltkreis 41 vorgesehen. Bei Stromausfall wird durch diesen ein akustisches Warnsignal und ein Rücksetzen des ersten R-S-Flip-Flops 38 ausgelöst, so daß die Ballonmanschette 3 entlüftet und der Respirator 44 umgeschaltet wird. Weiterhin bewirkt dieser Stromausfall, daß der Respirator 44 ausfällt und die Beatmung durch das am Respirator 44 für diesen Fall vorgesehene Handventil manuell erfolgt.

Hierbei wird die Atemgaszufuhr bzw. Abführung durch ein handbetätigtes Zweiwegeventil 42 mit inaktiver Mittelstellung vorgenommen, bis die Störung beseitigt oder ein anderes Beatmungsgerät angeschlossen ist.

**Patentansprüche**

1. Vorrichtung zur Erfassung und Auswertung des Druckes in der Ballonmanschette (3) eines geschlossenen Trachealtubus (1), mit einem an die Aufblaskanüle (6) der Ballonmanschette (3) angeschlossenen Druckwandler (10), dem ein Anzeigeinstrument (12) nachgeschaltet ist, und mit einer Steuerschaltung zum Entblocken der Ballonmanschette beim Überschreiten eines zulässigen Druckbereichs, dadurch gekennzeichnet, daß der Druckwandler ein Druck/Spannungs-Wandler (10) ist, dessen Ausgangssignal einerseits an einen ersten Grenzwertschalter (23) abgegeben wird, der beim Unterschreiten einer vorherbestimmten einstellbaren Schaltschwelle anspricht und eine Warnvorrichtung (25, 26) auslöst und andererseits an ein Differenzierglied (27) abgegeben wird, das über einen Doppelweg-Gleichrichter (29) und einen ersten Integrator (30) mit einem zweiten einstellbaren Grenzwertschalter (32) verbunden ist, der sowohl beim Überschreiten eines oberen vorherbestimmten Grenzwertes als beim Unterschreiten eines unteren vorherbestimmten Grenzwertes anspricht und über einen zweiten Integrator (36) an die Steuerschaltung (9, 14 bis 22, 37, 38) zum Entblocken der Ballonmanschette (3) angeschlossen ist.

2. Vorrichtung nach Anspruch 1, dadurch gekennzeichnet, daß den Grenzwertschaltern (23, 32) Grenzwertsteller (24, 33, 34) zugeordnet sind.

3. Vorrichtung nach Anspruch 1, dadurch gekennzeichnet, daß der zweite Grenzwertschalter zusätzlich eine Warnvorrichtung (35, 26) auslöst.

4. Vorrichtung nach Anspruch 1 oder 3, dadurch gekennzeichnet, daß die Ausgänge der Grenzwertschalter (23, 32) jeweils mit einer optischen Anzeigeeinrichtung (25, 35) und über ein ODER-Gatter (43) mit einer akustischen Anzeigeeinrichtung (26) verbunden sind.

5. Vorrichtung nach Anspruch 1, dadurch gekennzeichnet, daß der Ausgang des Druck/Spannungs-Wandlers (10) und der Ausgang des ersten Integrators (30) jeweils mit einem Druck-Anzeigeinstrument (31) verbunden sind.

6. Vorrichtung nach Anspruch 1, dadurch gekennzeichnet, daß die Steuerschaltung ein erstes R-S-Flip-Flop (14) aufweist, das zum Schließen des Absperrventils (9) in der Leitung zur Aufblaskanüle (6) über einen ersten Taster (13) sowie zum Öffnen des Absperrventils (9) über einen zweiten Taster (18) und über ein zweites R-S-Flip-Flop (38) steuerbar ist, das durch das Ausgangssignal des zweiten Integrators (36) von einem ersten durch einen dritten Taster (39) einschaltbaren der Dauerbeatmung zugeordneten Zustand in einen zweiten Zustand umschaltbar ist.

7. Vorrichtung nach Anspruch 6, dadurch gekennzeichnet, daß die Ausgänge des ersten R-S-Flip-Flops (14) an optische Statusanzeigeeinrichtungen (15, 20) angeschlossen sind.

8. Vorrichtung nach Anspruch 6 oder 7, dadurch gekennzeichnet, daß das Ausgangssignal des zweiten Integrators (36) sowie das Ausgangssignal eines Betriebsstromüberwachungsschaltkreises (41) über ein ODER-Gatter (37) mit einem Eingang des zweiten R-S-Flip-Flops (38) verbunden sind.

**Claims**

1. A device for measuring and evaluating the pressure in the balloon cuff (3) of a closed tracheal tube (1), having a pressure converter (10) which is connected to an inflation channel (6) of the balloon cuff (3), with an indicating instrument (12) being connected downstream of the converter, and having a controller to clear the balloon cuff if the permitted pressure range is exceeded, characterised in that the pressure converter is a pressure-voltage converter (10), the output signal of which is passed both to a first threshold value switch (23), which reacts when it falls below a previously determined and regulatable switching threshold and triggers a warning device (25, 26), and to a differentiator (27) which is connected via a two-way diode (29) and a first integrator (30) to a second regulatable threshold switch (32), which reacts both when an upper predetermined threshold value is exceeded and when a lower predetermined threshold value is crossed, and which is connected via a second integrator (36) to the controller (9, 14 to 22, 37, 38) for clearing the balloon cuff (3).

2. A device according to claim 1, characterised in that threshold value regulators (24, 33, 34) are assigned to the threshold value switches (23, 32).

3. A device according to claim 1, characterised in that the second threshold value switch additionally triggers a warning device (35, 26).

4. A device according to claim 1 or 3, characterised in that the outputs of the threshold value switches (23, 32) are each connected to a visual indicating apparatus (25, 35) and to an acoustic indicating apparatus (26) via an OR gate (43).

5. A device according to claim 1, characterised in that the output of the pressure-voltage converter (10) and the output of the first integrator (30) are each connected to a pressure-indicating instrument (31).

6. A device according to claim 1, characterised in that the controller has a first RS flip-flop (14) which can be controlled by means of a first scanner (13), for closing the cutout valve (9) in the pipe to the inflating channel (6), and by means of a second scanner (18) and a second RS flip-flop (38) for opening the cutout valve (9), the second RS flip-flop being switchable, by means of the output signal from the second integrator (36), from a first state to a second state, the first state being assigned to constant respiration and being activatable by a third scanner (39).

7. A device according to claim 6, characterised in that the outputs of the first RS flip-flop (14) are connected to a visual status-indicating apparatus (15, 20).

8. A device according to claim 6 or 7, characterised in that the output signal of the second integrator (36) and the output signal of an operating current supervisory switch circuit (41) are connected via an OR gate (37) to an input of the second RS flip-flop (38).

## Revendications

1. Dispositif de mesure et d'évaluation de la pression dans la manchette de ballon (3) d'un tube trachéal fermé (1), avec un convertisseur de pression (10) qui est raccordé à la canule d'insufflation (6) de la manchette de ballon (3) et à la suite duquel est monté un instrument d'affichage (12), et avec un circuit de commande pour débloquer la manchette de ballon lors du dépassement d'une plage de pression admissible, caractérisé en ce que le convertisseur de pression est un convertisseur de pression en tension (10), dont le signal de sortie est délivré d'une part à un premier commutateur à valeur limite (23) qui réagit lors du passage en dessous d'une valeur de seuil réglable prédéterminée et déclenche un dispositif d'alarme (25, 26), et est délivré d'autre part à un élément différenciateur (27) qui, par l'intermédiaire d'un redresseur biplaque (29) et d'un premier intégrateur (30), est relié à un deuxième commutateur à valeur limite (32) réglable qui réagit tant lors du dépassement d'une valeur limite supérieure prédéterminée que lors du passage en dessous d'une valeur limite inférieure prédéterminée, et qui par l'intermédiaire d'un deuxième intégrateur (36) est raccordé au circuit de commande (9, 14 à 22, 37, 38) pour débloquer la manchette de ballon (3).

2. Dispositif selon la revendication 1, caractérisé en ce que des régleurs de valeur limite (24, 33, 34) sont associés aux commutateurs à valeur limite (23, 32).

3. Dispositif selon la revendication 1, caractérisé en ce que le deuxième commutateur à valeur limite déclenche en outre un dispositif d'alarme (35, 26).

4. Dispositif selon la revendication 1 ou 3, caractérisé en ce que les sorties des commutateurs à valeur limite (23, 32) sont chacun reliés à un dispositif d'affichage optique (25, 35) et, par l'intermédiaire d'une porte OU (43), à un dispositif d'affichage sonore (26).

5. Dispositif selon la revendication 1, caractérisé en ce que la sortie du convertisseur de pression en tension (10) et la sortie du premier intégrateur (30) sont chacune reliées à un instrument d'affichage de pression (31).

6. Dispositif selon la revendication 1, caractérisé en ce que le circuit de commande présente une première bascule électronique RS (14), qui peut être commandée par l'intermédiaire d'une première touche (13) pour fermer la valve d'isolement (9) dans la conduite menant à la canule d'insufflation (6), et par l'intermédiaire d'une deuxième touche (18) et d'une deuxième bascule RS (38) pour ouvrir la valve d'isolement (9), ladite deuxième bascule pouvant, par le signal de sortie du deuxième intégrateur (36), être commutée d'un premier état, enclenchable par une troisième touche (39) et associé à la respiration continue, à un deuxième état.

7. Dispositif selon la revendication 6, caractérisé en ce que les sorties de la première bascule RS (14) sont raccordées à des dispositifs optiques d'affichage d'état (15, 20).

8. Dispositif selon la revendication 6 ou 7, caractérisé en ce que le signal de sortie du deuxième intégrateur (36) ainsi que le signal de sortie d'un circuit de surveillance du courant de service (41) sont reliés par l'intermédiaire d'une porte OU (37) à une entrée de la deuxième bascule RS (38).

$U_{Batt}$  $V_{DD}$

RESPIRATOR

Vakuum

$O_2 | N_2O$